# EUROPEAN PATENT SPECIFICATION

(11) **EP 3 632 468 B1**
(45) Date of publication and mention of the grant of the patent: **13.12.2023**
(21) Application number: 19200633.6
(22) Date of filing: 30.09.2019
(51) Int. Cl.: A61K 41/00, A61P 35/00

(54) **5-IODO-4-THIO-2'-DEOXYURIDINE FOR USE AS A RADIOSENSITIZER IN RADIOTHERAPY**
5-IOD-4-THIO-2'-DEOXYURIDIN ZUR VERWENDUNG ALS RADIOSENSIBILISATOR IN DER RADIOTHERAPIE
5-IODO-4-THIO-2'-DÉSOXYURIDINE POUR UNE UTILISATION COMME RADIOSENSIBILISATEUR EN RADIOTHÉRAPIE

(30) Priority: 02.10.2018 PL 42732618
(43) Date of publication of application: 08.04.2020
(73) Proprietor: Uniwersytet Gdanski, 80-309 Gdansk (PL)
(72) Inventor: Kozak, Witold, 83-000 Pruszcz Gdanski (PL); Rak, Janusz, 80-353 Gdansk (PL); Romanowska, Samanta, 80-398 Gdansk (PL); Spisz, Paulina, 80-288 Gdansk (PL); Zdrowowicz, Magdalena, 80-176 Gdansk (PL)
(74) Representative: Pawlowska, Justyna

(56) References cited:
- SAMANTA MAKURAT ET AL: "5-Iodo-4-thio-2'-Deoxyuridine as a Sensitizer of X-ray Induced Cancer Cell Killing", INT. J. MOL. SCI., vol. 20, no. 6, 1 March 2019 (2019-03-01), page 1308, XP055669266, ISSN: 1661-6596, DOI: 10.3390/ijms20061308
- PAULINA SPISZ ET AL: "Why Does the Type of Halogen Atom Matter for the Radiosensitizing Properties of 5-Halogen Substituted 4-Thio-2'-Deoxyuridines?", MOLECULES ONLINE, vol. 24, no. 15, 2 August 2019 (2019-08-02), page 2819, XP055669315, DE ISSN: 1433-1373, DOI: 10.3390/molecules24152819
- THEODORE L. PHILLIPS ET AL: "Results of a randomized comparison of radiotherapy and bromodeoxyuridine with radiotherapy alone for brain metastases: Report of RTOG trial 89-05", INTERNATIONAL JOURNAL OF RADIATION: ONCOLOGY BIOLOGY PHYSICS., vol. 33, no. 2, 1 September 1995 (1995-09-01), pages 339-348, XP055669309, USA ISSN: 0360-3016, DOI: 10.1016/0360-3016(95)00168-X
- C. J. MCGINN ET AL: "Radiosensitizing Nucleosides", JOURNAL OF THE NATIONAL CANCER INSTITUTE, vol. 88, no. 17, 4 September 1996 (1996-09-04), pages 1193-1203, XP055669312, GB ISSN: 0027-8874, DOI: 10.1093/jnci/88.17.1193
- BREM RETO ET AL: "UVA photoactivation of DNA containing halogenated thiopyrimidines induces cytotoxic DNA lesions", JOURNAL OF PHOTOCHEMISTRY AND PHOTOBIOLOGY B: BIOLOGY, ELSEVIER SCIENCE S.A., BASEL, CH, vol. 145, 20 February 2015 (2015-02-20), pages 1-10, XP029146401, ISSN: 1011-1344, DOI: 10.1016/J.JPHOTOBIOL.2015.02.012

## Description

The invention refers to an increase in the effectivity of radiotherapy by the use of radiosensitizer. The invention concerns the radiosensitizer of DNA damage, 5-iodo-4-thio-2'-deoxyuridine, for medical use in cancer radiotherapy.

Radiotherapy is the main method of cancer treatment. It uses ionizing radiation, which leads to cancer cell death. Unfortunately, the irradiation is not neutral to normal cells, causing a number of side effects. In addition, the solid cancer cells are characterized with hypoxia, which reduces the effectiveness of radiotherapy. Therefore, methods increasing the efficacy of radiotherapy and reducing its side effects are being searched. The efficient solution of this problem could be use of radiosensitizers, chemical compounds that sensitize cancer cells to ionizing radiation. Possible DNA radiosensitizers are compounds which produce DNA damage leading to cancer cell death when incorporated into DNA.

Phillips et al. disclose bromodeoxyuridine for use in radiotherapy (Phillips, Theodore L., et al. "Results of a randomized comparison of radiotherapy and bromodeoxyuridine with radiotherapy alone for brain metastases: Report of RTOG trial 89-05." International Journal of Radiation Oncology; Biology Physics 33(2) (1995): 339-348).

McGinn et al. disclose nucleosides for use in radiotherapy (McGinn, Cornelius J., et al., "Radiosensitizing Nucleosides." Journal of the National Cancer Institute 88(17) (1996): 1193-1203).

5-iodo-4-thio-2'-deoxyuridine (ISdU) is a compound with the structure characterized by X-ray crystallography, NMR, IR and MS techniques (Zhang, Xiao-Hui, et al. "5-iodo-4-thio-2'-deoxyuridine: synthesis, structure, and cytotoxic activity." Chemistry Letters 44.2 (2014): 147-149; Zhang, Xiao-Hui, et al. "NMR and UV Studies of 4-Thio-2'-deoxyuridine and Its Derivatives." Molecules 16.7 (2011): 5655-5664). The chemical method of ISdU synthesis is also known (Zhang, Xiao-Hui, et al. "5-iodo-4-thio-2'-deoxyuridine: synthesis, structure, and cytotoxic activity." Chemistry Letters 44.2 (2014): 147-149). The cytotoxic activity on human oral cancer cells of the CA-1 line has been described. The results show that ISdU can sensitize human kidney embryonic cells, of the HEK293 line, to UVA radiation. The synergic effect of ISdU and UVA radiation on mouse and human fibroblasts and human cervical cancer cells has been also observed. The use of ISdU/UVA leads to the formation of DNA damage like interstrand crosslinks and DNA-protein crosslinks. Moreover, ISdU can be incorporated into DNA (Brem, Reto, et al. "UVA photoactivation of DNA containing halogenated thiopyrimidines induces cytotoxic DNA lesions." Journal of Photochemistry and Photobiology B: Biology 145 (2015): 1-10; Brem, Reto, et al. "Oxidatively-generated damage to DNA and proteins mediated by photosensitized UVA." Free Radical Biology and Medicine 107 (2017): 101-109). ISdU possesses strong antiviral activity against e.g. orthopediviruses and herpesviruses (Prichard, Mark N., et al. "Inhibition of herpesvirus replication by 5-substituted 4'-thiopyrimidine nucleosides." Antimicrobial agents and chemotherapy 53.12 (2009): 5251-5258; Keith, Kathy, et al. "Combinations of 5-iodo-4'-thio-2'-deoxyuridine and St-246 or Cmx001 Synergistically Inhibit Orthopoxvirus Replication In Vitro: 58." Antiviral Research 82.2 (2009): A33-A34). Studies on stationary photolysis of deoxygenated aqueous solution of ISdU and flash photolysis showed that the irradiation of ISdU with UV radiation of 334 nm leads to a generation of stable photoproducts SdU, IdU-S-S-IdU. A mechanism of this photochemical process has been also proposed (Weńska, Grażyna, et al. "Generation of thiyl radicals by the photolysis of 5-iodo-4-thiouridine." The Journal of organic chemistry 70.3 (2005): 982-988).

Any references to methods of treatment in this description are to be interpreted as references to the compounds, pharmaceutical compositions and medicaments of the present invention for use in a method for treatment of the human (or animal) body by therapy.

The invention concerns a medical use of 5-iodo-4-thio-2'-deoxyuridine as a DNA radiosensitizer - a substance making cancer cells more susceptible to ionizing radiation in radiotherapy, especially in solid tumors. The chemical structure of this compound is presented in Formula 1. The term "DNA radiosensitizer" refers to the fact that the compound enhances the efficacy of damage to genomic DNA leading to subsequent cellular death. The results of the research carried out on the compound, which are described in the examples below, confirm the use of ISdU in radiotherapy, especially against solid tumors, because hypoxia, that is employed in sensitization according to the invention, is characteristic for this kind of tumors. Hence, the results confirm effectiveness of medical use of ISdU, according to the invention, in radiotherapy with X-ray and/or gamma radiation.

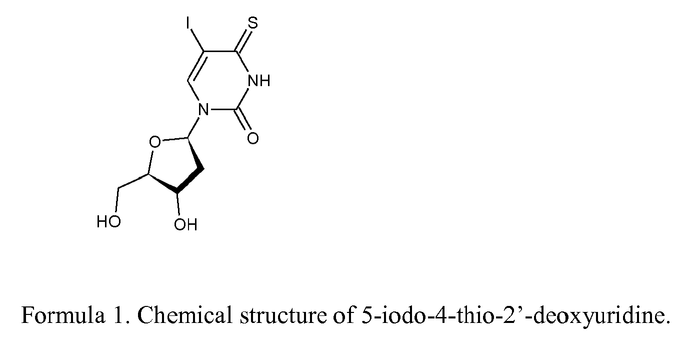

The invention is described in details in the examples and in drawings wherein fig. 1 shows a graph that depicts the decrease in the survival of MCF-7 breast cancer cells in the function of radiation dose and ISdU concentration, fig. 2 shows cytotoxicity of ISdU against human MCF-7 breast cancer cell line, fig. 3 depicts the cytometric analysis of phosphorylation of H2A.X histone, while Scheme 1 shows possible reaction pathways leading to products of radiolysis of ISdU.

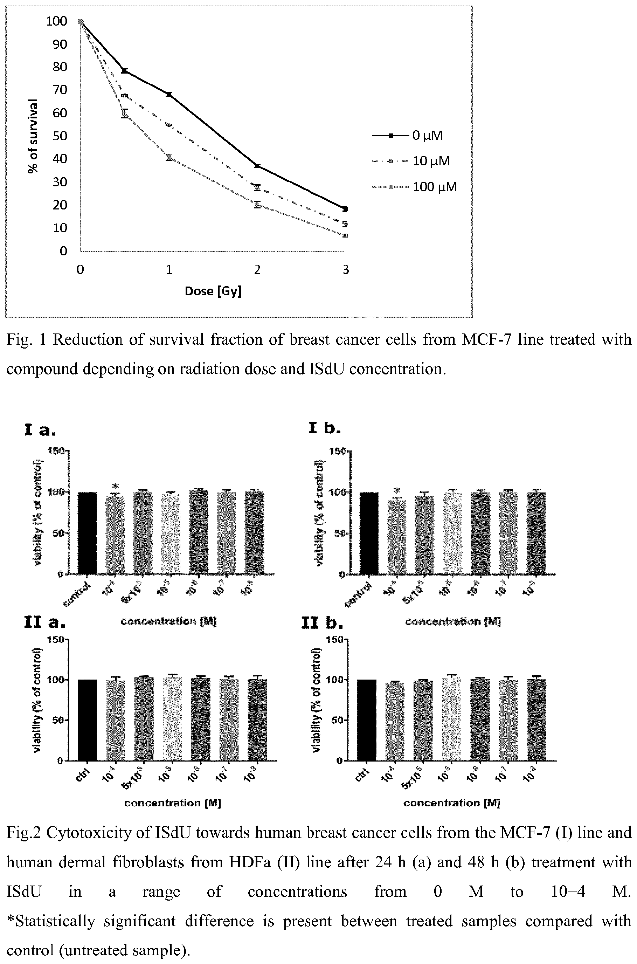

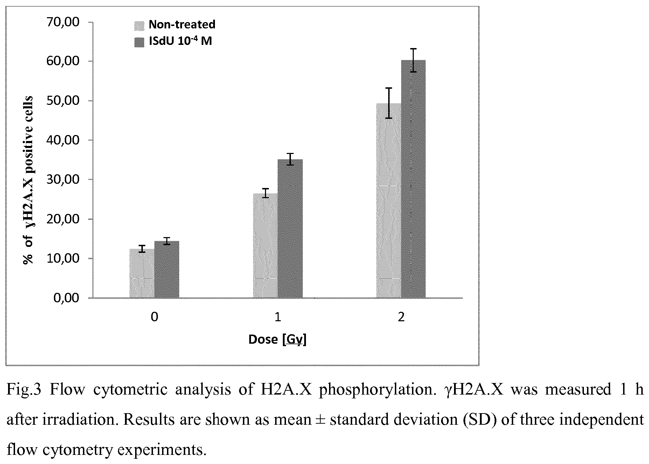

It has been proved, that ISdU sensitizes MCF-7 cancer cells to ionizing radiation. The invention concerns the effective radiosensitizer, which enhances the efficiency of radiotherapy and allows to reduce the radiation dose, which is strictly connected to a better radiologic protection of healthy tissues. The conducted clonogenic assays prove that treatment of breast cancer cells with ISdU results in the decrease in survival after irradiation with such low doses as 1.0 or even 0.5 Gy, which is showed in fig. 1. Due to the fact that the compound itself possesses low cytotoxicity, undesirable effects in healthy cells are not observed, which is shown in fig. 2. Moreover, the research shows that ISdU undergoes a degradation triggered by the attachment of solvated electrons, that arise in the cancer cell - characterized by the diminished oxygen level - in the same amount as highly genotoxic hydroxyl radicals, which is shown in Scheme 1. This means the increase in the efficacy of cancer cell death (containing ISdU-labeled DNA) induced with ionizing radiation. Besides, in order to evaluate the efficiency of the compound, according to the invention, cytometric analysis of phosphorylation of histone H2A.X - the molecular marker of double-strand breaks - was performed. These results are shown in the example 4 and in fig. 3.

ISdU was obtained with use of previously described methods - a four-step chemical synthesis (Zhang, Xiao-Hui, et al. "5-iodo-4-thio-2'-deoxyuridine: synthesis, structure, and cytotoxic activity." Chemistry Letters 44.2 (2014): 147-149). In the first step, 3'- and 5'-hydroxyl present in the sugar moiety were acyl-protected *via* the reaction with acetic anhydride in pyridine. Next, an iodine atom was introduced into the constitution of acetyl-protected 2'-deoxyuridine with use of molecular iodine and a Ce(IV) derivative. The third step comprised the substitution of oxygen atom with sulfur atom in the presence of P₂S₅. Finally, the hydroxyl groups were deprotected with use of the methanolic solution of sodium methanolate, generated *in situ* in the reaction of methanol with sodium hydride.

### Example 1

Decrease in survival of cancer cells after exposure to ionizing radiation as a result of incubation of the culture with ISdU.

Clonogenic assays were used to assess the survival and proliferation potential of MCF-7 human breast cancer cells treated with ISdU and/or IR.

MCF-7 human breast cancer cells were plated at a density of 10⁶ cells per 60 mm dish. The cells were treated with ISdU in concentrations of 0, 10 and 100 µM. The cells were grown in RPMI medium supplemented with 10% fetal bovine serum (FBS) and antibiotics (streptomycin and penicillin) at a concentration of 100 U·mL⁻¹. After 48 h of treatment, the cells were irradiated with dose 0-3 Gy using CellRad X Ray Chamber, Faxitron X-ray Corporation. After 6 h the cells were trypsynized, plated at a density of 800 cells per 100 mm dish and incubated under 37°C, 5% CO₂. After 16 days the resulting colonies were fixed with 6.0% (v/v) glutaraldehyde and 0.5% crystal violet. Stained colonies were counted manually, and colony size was assessed using an inverted fluorescence microscope e.g. Olympus, IX73. The experiment was carried out in duplicate. Non-irradiated cultures were used as controls.

The performed clonogenic assay proved that the treatment of breast cancer cell with 10 µM ISdU resulted in a significant decrease even about 16 % of their survival after irradiation, with doses as low as 1 Gy in comparison to non-irradiated control. The survival of cells, pre-treated with 100 µM solution of ISdU and exposed to 1 Gy of IR, was reduced by about 30%.

The results are shown in Fig. 1 - a graph showing a decrease in the survival of MCF-7 breast cancer cells as a result of treatment of the culture with the tested compound.

### Example 2

Studies on the cytotoxicity of ISdU towards human breast cancer cells - MCF-7 line and human dermal fibroblasts - HDFa line.

Cytotoxic activity was determined using the MTT assay after incubation of MCF-7 (CLS, Eppelheim) or HDFa (Gibco Invitrogen) cells with ISdU. The MCF-7 cells were grown in RPMI supplemented with 10% FBS and antibiotics (streptomycin and penicillin) at a concentration of 100 U·mL⁻¹. The HDFa cells were grown in DMEM supplemented with 10% FBS and antibiotics (streptomycin and penicillin) at the same concentration. Cells at a density of 4·10³ per well were seeded into a 96-well plate and incubated (37°C, 5% CO₂) for 24 h. Then, the medium was freshly changed, and the cells were treated with ISdU at concentrations of 0 (control), 10⁻⁴, 5·10⁻⁵, 10⁻⁵, 10⁻⁶, 10⁻⁷ and 10⁻⁸ M. Cells were incubated with ISdU for 24 h and 48 h. After this time, the aqueous MTT salt solution at a concentration of 4 mg·mL⁻¹ was added to each well. After 4 h of incubation, the medium was removed and 200 µL of dimethylsulfoxide (DMSO) was added to dissolve the formazan crystals. The absorbance was measured at 570 nm (with 660 nm taken as the reference) on an EnSpire microplate reader (PerkinElmer). The data was obtained from three independent experiments and each treatment condition assayed in triplicate. The results were analyzed with the use of GraphPad Prism software. The statistical evaluation of treated samples and untreated controls was calculated using one-way analysis of variance (ANOVA) followed by Geisser-Greenhouse's multiple comparison test. The differences were considered significant at P < 0.05.

The compound is non-cytotoxic towards both normal and studied cancer cells lines. The pre-treatment with 100 ISdU µM caused a statistically significant reduction in MCF-7 cells viability compared to the control, to 91 ± 3% for 48 h incubation. In case of HDFa cells, a statistically significant decrease in cell viability was not observed.

The results, which are shown in Fig. 2, depict the effect of ISdU on the viability of human breast cancer cells from the MCF-7 line and healthy human breast fibroblasts from the HDFa line.

### Example 3

Electron induced degradation of 5-iodo-4-thio-2'-deoxyuridine.

Mechanisms of reactions occurring during ISdU derivative radiolysis were explained using quantum-chemical calculations at the level of density functional theory (DFT). All calculations were performed with the use of the B3LYP functional and the DGDZVP++ basis sets. In order to simplify the calculations, we substituted the 2'-deoxyribose residue, which is not involved in the IR-induced damage, with a methyl group (5-iodo-1-methyl-4-tiouracil, ISU, Scheme 1). The water environment was simulated with the use of the polarizable continuum model (PCM) approach. All calculations were carried out with the Gaussian09 package. In addition, according to the literature suggestions, in the case of oxidation reactions, *explicit* water (significant transient stabilization due to better charge distribution, impossible to obtain in a simplified PCM model) was included. It turned out that the radiolysis of the studied derivative, caused by X-ray irradiation, results in the generation of five major radioproducts: SU, two oxidation products ISOU and IU and two types of dimers - IU-S-S-IU and U-S-S-IU in Scheme 1 (in case of ISdU, it would be SdU, ISOdU, IdU, IdU-S-S-IdU and dU-S-S-IdU, respectively).

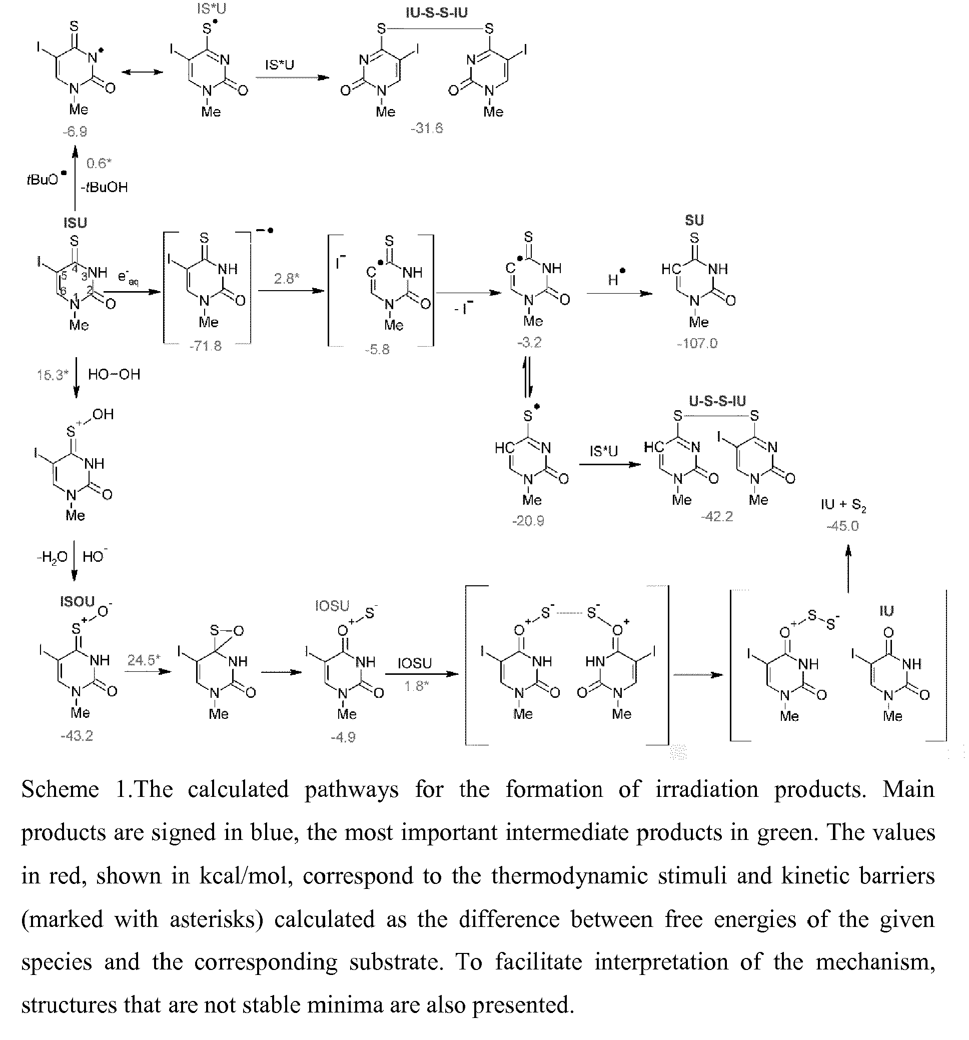

Products resulting from ISdU radiolysis use both the solvated electron (e_{aq}-) and other individuals present in the solution, especially hydrogen peroxide (H₂O₂) and the t-butyl radical (*t*BuO^{•}). The first two: e_{aq}- and H₂O₂, are formed during water radiolysis (a well-known water radiolysis product resulting from recombination of ^{•}OH radicals), and the last one, *t*BuO^{•}, is formed from a free radical scavenger - *t*-butanol, added to the solution in a large excess.

It has been confirmed that the described compound sensitizes breast cancer cells (MCF-7 line) to ionizing radiation. The clonogenic assay proved that the survival of cells, pre-treated with 10 or 100 µM solution of ISdU and exposed to 1 Gy of IR, is reduced by approximately 16% - 10 µM solution of ISdU and approximately 30% - 100 µM solution of ISdU. unless not exposed to IR, possesses low cytotoxicity.

ISdU is effectively degraded in an aqueous solution by X-ray irradiation. The quantum-chemical studies show a variety of products and paths on which they are formed, which, in turn, indicates the potential of ISdU to be used as DNA radiosensitizer.

### Example 4

One of the damages caused by ISdU are double strand break of DNA, whose molecular marker is phosphorylation of histone H2A.X. To confirm the application of the compound in radiotherapy, the cytometric analysis of phosphorylation of histone H2A.X, molecular marker of double strand break of DNA was carried out.

The MCF-7 cells (human breast cancer cells) were grown up in RPMI medium supplemented with 10 % FBS (fetal bovine serum) and antibiotics (penicillin and streptomycin) at a concertation of 100 U · ml⁻¹. The cells were treated with ISdU at the concentration of 10⁻⁴ M and incubated (37°C, 5% CO₂) for 48 h. Upon this time, plate cultures were X-ray irradiated with doses of 1 Gy and 2 Gy, using of e.g. Cellrad X-ray cabinet, Faxitron X-ray Corporation, and incubated for 1 h in the same condition. Next, the cells were detached with solution of Accutase (Life Technologies, UK), fixed and permeabilized (fixation and permeabilization was performed by the manufacturer's protocol FlowCellect^{™} Histone H2A.X Phosphorylation Assay Kit, Merck). Prepared cells were analyzed by cytometric using of e.g. Guava easyCyte^{™}, Merck. The experiment was performed in triplicated. The non-treated cells were a control.

The results showed that the treating of cells with ISdU after irradiation with the 1 and 2 Gy doses lead to increase of γH2A.X positive cells (cells showing phosphorylation of histone H2A.X). For dose of 1 Gy the increase of γH2A.X level from 26.56 ± 1.14% (for control) to 35.15 ± 1.48%, while for dose of 2 Gy from 49.38 ± 3.8% to 60.25 ± 2.98% was observed.

The results presented in fig. 3, show an assessment of histone H2A.X phosphorylation level performed by using flow cytometry for the MCF-7 cells. The level of γH2A.X was measured 1 h after the irradiation. The results suggested the increase of double strand breaks after using the compound. It directly correlates with the death of cells.

## Claims

1. Radiosensitizer in the form of 5-iodo-4-thio-2'-deoxyuridine for use in radiotherapy.

2. The radiosensitizer for use according to claim 1 in the treatment of solid cancers.

3. The radiosensitizer for use according to claim 1 or 2 in radiotherapy using X and / or gamma rays.

## Patentansprüche

1. Der Radiosensitizer in Form von 5-iodo-4-thio-2'-deoxyuridine zur Anwendung in der Radiotherapie.

2. Der Radiosensitizer zur Anwendung gemäß Anspruch1 bei der Behandlung von soliden Krebserkrankungen.

3. Der Radiosensitizer zur Anwendung gemäß Anspruch1 oder 2 in der Radiotherapie mit Röntgen- und/oder Gammastrahlen.

## Revendications

1. Radio sensibilisateur sous forme de 5-iodo-4-thio-2'-désoxyuridine pour utilisation en radiothérapie.

2. Radio sensibilisateur pour utilisation selon la revendication 1 dans le traitement des cancers solides.

3. Radio sensibilisateur pour utilisation selon la revendication 1 ou 2 dans la radiothérapie utilisant les rayons X et/ou gamma.
